# EUROPEAN PATENT SPECIFICATION

(11) **EP 2 544 595 B1**
(45) Date of publication and mention of the grant of the patent: **06.01.2016**
(21) Application number: 11707014.4
(22) Date of filing: 23.02.2011
(51) Int. Cl.: A61B 10/02

(54) **NEEDLE WITH HELICAL GROOVES CONVERTING AXIAL MOVEMENT TO ROTATIONAL MOVEMENT**
NADEL MIT SPIRALFÖRMIGEN KERBUNGEN ZUR UMWANDLUNG EINER AXIALBEWEGUNG IN EINE DREHBEWEGUNG
AIGUILLE DOTÉE DE RAINURES HÉLICOÏDALES CONVERTISSANT UN MOUVEMENT AXIAL EN UN MOUVEMENT ROTATIF

(30) Priority: 10.03.2010 US 312374 P
(43) Date of publication of application: 16.01.2013
(73) Proprietor: Boston Scientific Scimed, Inc., Maple Grove, MN 55311-1566 (US)
(72) Inventor: CARRILLO, JR., Oscar R., Attleboro Massachusetts 02703 (US); DEVRIES, Robert B., Northborough Massachusetts 01532 (US); GOLDEN, John, Norton Massachusetts 02766 (US); GRIEGO, John A., Blackstone Massachusetts 01504 (US); SHAW, William J., Cambridge Massachusetts 02138 (US)
(74) Representative: Vossius & Partner Patentanwälte Rechtsanwälte mbB
(86) International application number: PCT/US2011/025844
(87) International publication number: WO 2011/112357

(56) References cited:
- EP-A1- 1 829 571
- WO-A2-2009/079155
- US-A1- 2001 005 778
- US-A1- 2006 116 605

## Description

### Background

Needle catheters are often employed to inject medication into a body, obtain fluid and/or tissue samples, etc. In these procedures, a needle is advanced to a target tissue site guided using endoscopic vision systems or other imaging techniques (e.g., ultrasound). The needle is then advanced distally from the catheter to penetrate the target location. In biopsy procedures, suction is then applied (e.g., via a syringe) to draw desired sample tissue into the needle. During this process, the needle may be moved back and forth repeatedly to harvest the desired sample, which is drawn through the needle to a proximal end thereof for analysis. In these cases, tissue trauma is increased due to the increased friction between the needle catheter and the skin and intervening tissue.

### Summary of the Invention

The present invention is directed to a device for penetrating tissues within a living body, comprising a shaft extending from a proximal end which, in an operative position, remains outside the body to a distal end which, in the operative position, is within a living body the shaft comprising a working channel extending therethrough from the proximal end to the distal end comprising an opening. The device also comprises a needle extending within the working channel, the needle comprising a tissue penetrating distal tip, a portion of an outer surface of the needle including a first structure extending along a path wrapping around a portion of a length of the needle, the first structure configured to mate with a second structure formed on a corresponding portion of an inner wall of the shaft, the first and second structures mating with one another so that, as the needle is urged axially through the working channel, the first and second structures cause the needle to rotate about an axis of the working channel.

### Brief Description of the Drawings

Fig. 1 shows a cross-sectional view of a distal end of a device according to a first embodiment of the present invention;
Fig. 2 shows a cross-sectional view of a distal end of a device according to a second embodiment of the present invention;
Fig. 3 shows a partially cross-sectional view of a proximal end of a device according to a third embodiment of the present invention;
Fig. 4 shows a perspective view of a device according to a fourth embodiment of the present invention; and
Fig. 5 shows a partial cross-sectional view of a distal portion of the device of Fig. 4.

### Detailed Description

The present invention, which may be further understood with reference to the following description and the appended drawings, relates to a device for rotating a biopsy needle within the body. It is noted that although the exemplary embodiment of the present invention is described with respect to particular procedures within a living body, the description is not meant to limit the application of the invention, which may be employed in any of a number of procedures requiring the insertion of a needle into a living body.

When performing a biopsy procedure, it is often desirable to screw the needle into the target tissue without coring. This motion allows tissue samples to be captured with less need for suction and the consequent drawing of blood and other non-targeted tissues into the needle. This motion, known as juicing, is generally performed by manually rotating the proximal end of the device to transmit the torque along the device shaft to rotate the needle at the distal end. Unfortunately, this rotation causes the shaft of the device to wind up (i.e., twist), reducing the rotation of the distal tip of the needle when compared to the degree of rotation of the proximal handle. Furthermore, rotating the entire needle catheter may cause pain and injury or result in whipping, as those skilled in the art will understand.

Devices and methods according to an exemplary embodiment of the present invention permit rotation of the distal tip of a needle without requiring rotation of the proximal end thereof. Specifically, the user applies to the device only axially directed forces (i.e., forces directed along a longitudinal axis of the device). A portion of this force is converted at the distal end of the device to a rotational force generating a desired rotation of a needle about the axis.

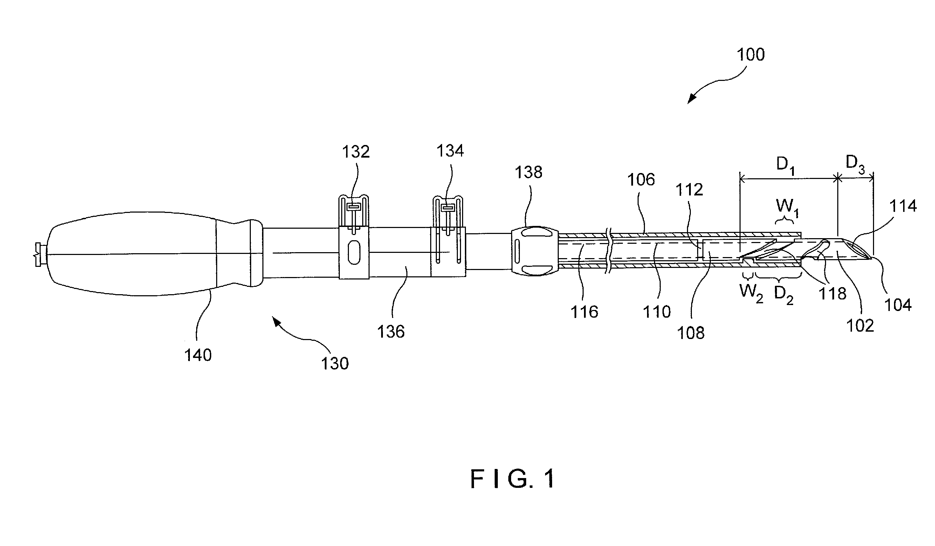

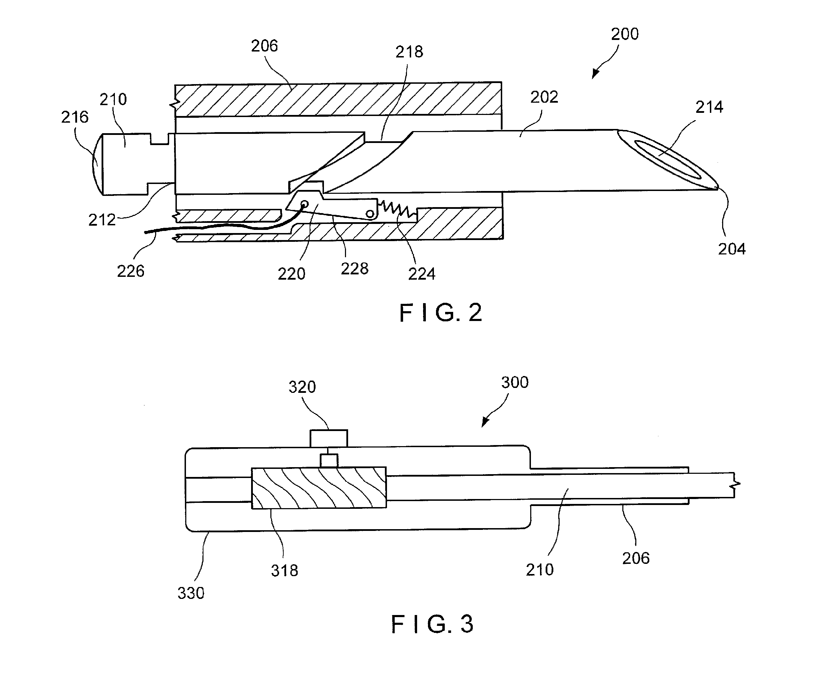

As shown in Figs. 1 and 2, a device 100 according to a first exemplary embodiment of the present invention includes a needle 102 comprising a puncturing tip 104 at a distal end thereof received within a catheter shaft 106. It is noted that the use of the term distal herein refers to a direction away from a user of the device while the term proximal refers to a direction approaching a user of the device. The proximal portion of the device 100 including a handle 130 remains external to the body accessible to the user while the distal portion, when in an operative position, extends into the body to a target site from which tissue samples are to be obtained.

A distal portion 108 of the needle 102 including the puncturing tip 104 is rotatably attached to a proximal shaft 110 via a rotating joint 112. The joint 112 may allows for any desired degree of rotation between the shaft 110 and the distal portion 108 of the needle 102 and preferably allows for an unlimited range of free rotation. As will be made clear later, any desired limits to the permitted degree of rotation of the distal portion 108 relative to the shaft 110 may be set through the construction of a mechanism for translating axial motion of the shaft 110 into axial and rotational movement of the distal portion 108. However, any desired limit to relative rotation between these parts may also be set through the construction of the joint 112. Furthermore, as would be understood by those skilled in the art, the joint 112 also preferably includes a lumen (not shown) extending therethrough to couple a lumen 114 of the distal portion 108 to a lumen 116 in the shaft 110. The lumen (not shown) thus permits samples to be drawn through the shaft 110 out of the body without removing the device 100 from the body. The lumen of the joint 112 is preferably coupled with the lumens 116 and 114 to provide a smooth unobstructed flow path for materials passed therethrough, thereby preventing any trauma, damage or flow resistance to said materials.

The needle 102 of the device 100 contains a groove 118 extending along a substantially helical path around a portion of an axial length of the needle 102. Specifically, the groove 118 spans a distance D₁ along the needle 102 and terminates at a predetermined distance from the distal end of the needle 102. In an exemplary embodiment, a pitch of the helical groove 118 is selected so that a predetermined amount of axial movement of the needle 102 causes a rotation of approximately 360°, although any other pitch may be employed without deviating from the scope of the present invention. It is noted that although the groove 118 is depicted as extending along only a predetermined distal length of the needle 102, in an alternate embodiment the groove 118 may extend for substantially the entire length of the needle 102. As will be described below in greater detail below, the groove 118 provides for the rotation of the needle 102 upon entry and withdrawal thereof from the catheter shaft 106. A width W₁ of the groove 118 is substantially equal to or slightly greater than a width W₂ of an abutment 120 located on the inner portion of the catheter shaft 106, the groove 118 providing clearance for the sliding of the abutment 120. A height of the abutment 120 relative to a depth of the groove 118 is preferably selected to prevent the removal of the abutment 120 from the groove 118 at any location other than a distal end of the groove 118. During insertion into and removal from the body and when moving within the body between separate target sites, the needle 102 is preferably maintained in a retracted state with the abutment 120 located in a distal most portion of the groove 118 and a pointed distal tip of the needle 102 housed within the catheter 106. Accordingly, a distance D₂ corresponding to an axial distance from a distal end of the abutment 120 to a distal end of the catheter 106 may be substantially equal to or greater than a distance D₃ corresponding to a length of the needle between a distal most portion of the groove 118 and the distal end of the puncturing tip 104. It is noted however, that the distance D₂ may also be smaller than the distance D₃ without deviating from the scope of the present invention. In such an embodiment, the groove 118 may be positioned proximally of the abutment 120 in an insertion configuration and may only be brought into contact with the abutment after the catheter shaft 106 has been brought to a target tissue site in the body so as to prevent trauma to untargeted tissue during insertion, as those skilled in the art will understand. In an alternate embodiment incorporating a cap or other structure to protect surrounding tissue from the puncturing point 104, the distance D₂ may be smaller than the distance D₃.

As the shaft 110 is moved axially in a distal direction via actuation of an actuator on the handle 130 and the needle 102 is extended distally from the catheter shaft 106, the abutment 120 is forced to slide along the groove 118. This causes the needle 102 to rotate (clockwise as viewed looking proximally) as it moves distally.

The abutment 120 also serves to prevent the needle 102 from being extended distally from the catheter 106 beyond a predetermined length. For example, the axial length D₁ of the groove 118 is preferably chosen to permit the needle 102 to protrude distally from the catheter shaft 106 only by a certain distance selected, for example, on the needs of the particular procedure being performed and the mechanical properties of the device 100, as those skilled in the art will understand. After a procedure has been completed, the user of the device 100 retracts the needle 102 by moving the proximal handle portion 140 and the first actuator along at central actuation portion 136 of the handle 130. Specifically, movement of the first actuator 132 in a proximal direction toward the proximal handle portion 140 causes retraction of at least a portion of the device 100 into the handle 130. Movement of the first actuator 132 distally away from the proximal handle portion 140 likewise generates distal movement of the needle 102 until the puncturing tip 104 of the needle 102 is fully exposed outside of the catheter 106. It is noted that the design of the handle 130 may take any desired shape as dictated, for example, by ergonomics, etc. and is not limited to the arrangement shown in the embodiment of Fig. 1. Furthermore, it is noted that the catheter 106 containing the lumen 116, the shaft 110 and the needle 102 may extend proximally from the handle 130 of the device 100 by any length, which length may, for example, be selected to conform to the specific requirements for a procedure being performed. The handle 130 is also configured to permit manual rotation of the device 100 if so desired. Specifically, the catheter 106 may be threadedly engaged with a connector 138 located at the end of the handle 130, the connector 138 being rotatable via manual rotation of a proximal handle portion 140, such rotation being translated only to a lumen (not shown) extending through the handle and to the connector 138 and device 100. Application of this rotational force to the device 100 is likewise converted to rotation of the needle 102, in the opposite direction (i.e., counterclockwise when looking proximally). When the groove 118 of the needle 102 engages the abutment 120, rotation of the needle 102 similarly causes a rotation of the connector 138 and the proximal handle portion 140 until the abutment 120 comes to rest in the distal end of the groove 118 and the puncturing tip 104 fully into the catheter 106.

This exemplary embodiment allows the rate of rotation and the orientation of the puncturing tip 104 of the needle 102 to be controlled to desired rates. Furthermore, as would be understood by those skilled in the art, by varying the pitch or helix angle of the groove 118 along its length, a ratio of the rotational speed of the catheter relative to the axial movement thereof may vary. For example, to ease the start of rotation of the needle 102, the helix angle may be reduced at both the proximal and distal ends of the groove 118 and, then steepen to speed the rotation of the needle as the abutment travels along a middle portion of the groove 118. In addition, since the abutment 120 engages the groove 118 with a substantially tight friction fit, undesired movement of the needle 102 is prevented, improving the accuracy of the movements of needle 102 and eliminating the need for a user of the device 100 to directly apply rotational forces to the proximal end to avoid problems associated therewith (e.g., wind up, whipping, etc.).

As shown in Fig. 2, a device 200 according to an alternate embodiment of the invention is functionally and structurally similar to that of the device 100 except for the differences detailed below. The device 200 comprises a catheter shaft 206 containing a needle 202 with a helical groove 218 formed therein to convert axial movement of a shaft 210 and the needle 202 into a combined rotary and axial motion in the same manner as above in regard to the device of Fig. 1. A lumen 216 in the shaft 210 is fluidly connected via a lumen (not shown) in the rotatable joint portion 212, to a lumen 214 extending through the needle 202. The needle 202 of the device 200 extends from the rotating joint 212 (e.g., a freely rotating joint) at a proximal end to a puncturing tip 204 located at a distal end thereof. The catheter shaft 206 houses therein a pawl 220 which engages with the helical groove 218 in substantially the same manner as the abutment 120 of the device of Fig. 1. Specifically, the pawl 220 engages the helical groove 218 to guide the rotary motion of the needle 202 into and out of the catheter shaft 206 and through the target tissue. The pawl 220 is rotatably coupled to the catheter 206 for movement between an engaged position in which the pawl 220 engages the groove 218 and a retracted position in which the pawl 220 is disengaged from the groove 218 and resides in a recess 228 formed in the wall of the catheter 206. The pawl 220 may, for example, be biased toward the engaged position by a spring 224 or other biasing mechanism as would be understood by those skilled in the art. In addition, a mechanism such as a pull wire 226 or a rod (not shown) coupled to an actuator on the handle (not shown) may be used to pull the pawl 220 into the disengaged position over the bias of the spring 224. This allows an operator to move the pawl 220 between the engaged and retracted positions so that, for example, the needle 202 may be rotated as it is extended and then the pawl 220 may be disengaged to allow the needle 202 to be retracted without rotating. In order to ensure that the pawl 220 is aligned with the groove 218 when the needle 202 is retracted without rotation, the distal end of the groove 218 and the proximal end of the groove 218 must be in substantially the same position around a circumference of the needle 202. In another embodiment of the present invention (not shown), a proximal wall of the pawl 220 may be ramped and the pawl 220 may be seated against the inner wall of the catheter shaft 206 without a recess 228.

As would be understood by those skilled in the art, the aforementioned operation may be automated using known mechanisms. For example, such a mechanism can be coupled to the actuator which applies axial force to the shaft 210 so that, when a user of the device 200 actuates the actuator to withdraw the needle 202 proximally, the pawl 220 is retracted into the recess 228 out of engagement with the groove 218 allowing the needle 202 to withdraw into the catheter shaft 206 without rotating. If the pawl 220 is allowed to spring back against the surface of the needle 202 after being withdrawn from the groove 218, proximal surfaces of portions of the groove 218 circumferentially aligned with the proximal and distal ends of the groove 218 may be ramped to allow the pawl 220 to ratchet therepast as the needle 202 is retracted proximally until the pawl 220 enters and locks into the proximal end of the groove 218. The device 200 thus minimizes rotation of the needle 202 through the tissue reducing trauma thereto.

Fig. 3 shows a proximal portion 300 of a device such as the device 200 permitting a user to selectively engage the pawl 220 with the groove 218 as described above. Specifically, a handle 330 includes a first actuator 318 which applies an axially directed force proximally and distally to the shaft 210 and the needle 202 while a second actuator 320 moves the pawl 220 between the engaged and disengaged positions. This arrangement allows a user to select a rotary needle motion for any portion or a complete duration of a procedure and/or a linear, non-rotational needle movement for any portion or a complete duration of another procedure. As would be understood by those skilled in the art, one or both of the actuators 318, 320 may include any known locking mechanism to maintain the actuator in a desired position.

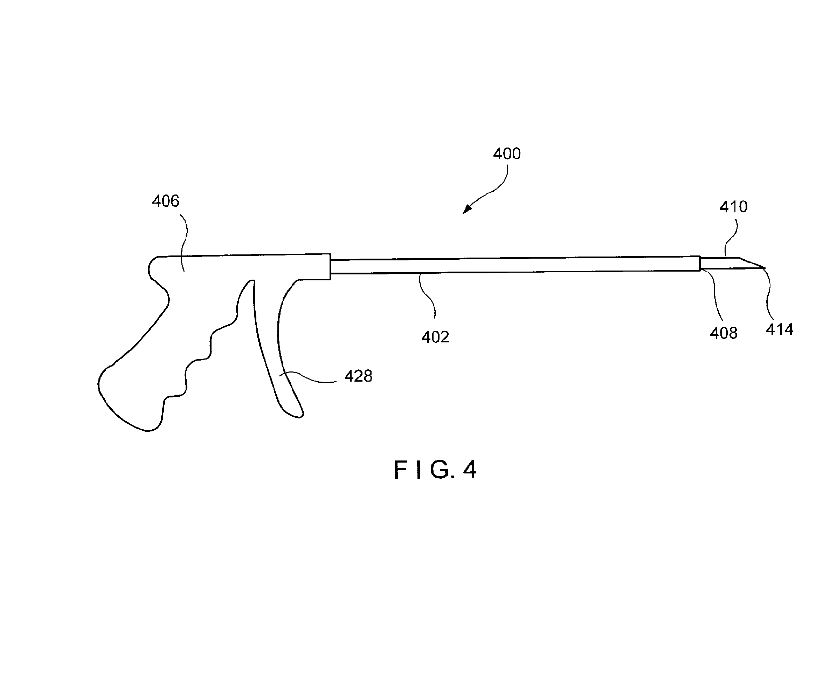

Figs. 4 - 5 depict a device 400 according to a fourth exemplary embodiment of the present invention, wherein the device 400 is formed substantially similarly as the device 100 of Fig. 1 with the exception of an advancing mechanism provided thereon and a handle. The device 400 comprises an outer member 402 including a working channel 404 extending from a handle 406 at a proximal end thereof to a distal opening 408. Received within the working channel 404 is a needle member 410 which extends from a proximal end mounted to an actuator 428 of the handle 406 to a tissue penetrating tip 414 at a distal end thereof. A selected portion 416 of the inner walls 418 of the outer member 402 defining the working channel 404 is shaped to correspond to a shape of an outer surface 420 of a corresponding portion of the needle member 410 received therein as will be described in more detail below. The needle member 410 also includes a lumen 422 extending therethrough to a distal opening 424 at the penetrating tip 414. As would be understood by those skilled in the art, an inner surface of the lumen 422 is preferably substantially smooth and free from bumps or ridges which may cause trauma to histological samples drawn thereinto.

As mentioned above, the selected portion 416 of the inner wall 418 is threaded to mate with a correspondingly threaded portion 426 of the needle member 410. The threading engagement of the portion 416 of the inner wall 418 and the portion 426 of the needle member 410 causes the needle member 410 to rotate about its longitudinal axis as it is moved axially through the outer member 402, wherein one or both of the inner wall 418 and the threaded portion 426 are lubricated to better encourage rotation. Since an inner diameter of a portion of the working channel 404 distal of the portion 426 of the needle member 410 is smaller than a maximum diameter of the portion 426, the position of the distal end of the portion 416 defines a maximum extent of projection of the needle member 410 beyond the distal end of the outer member 402. Similarly, since an inner diameter of a portion of the working channel 404 located proximally of the portion 426 of the needle member 410 is smaller than a maximum diameter of the portion 426, the position of the proximal end of the portion 416 defines a maximum extent of withdrawal of the needle member 410 proximally into the working channel 404.

Operation of an actuator 428 on the handle 406 urges the needle member 410 distally and withdraws it proximally substantially along a longitudinal axis of the outer member 402 - i.e., axially within the working channel 404. As would be understood by those skilled in the art, the actuator 428 may operate in the manner of any known mechanism for moving a needle axially through a working channel provided the needle member 410 is free to rotate about its longitudinal axis relative to the handle 406. In a preferred embodiment, an actuating mechanism for the needle member 410 may be manually driven by the actuator 428 wherein a reciprocal motion of the actuator 428 is translated to a swiveling proximal-distal movement of the needle member 410. As the needle member 410 moves proximally and distally through the working channel 404, the threaded engagement of the portion 416 of the inner wall 418 and the portion 426 of the needle member 410 causes the needle member 410 to rotate about its axis, causing a corresponding rotation of the distal end of the needle member 410 and the tissue penetrating distal tip 414. This allows the tip 414 to screw into the target tissue without puncturing or coring. This motion allows tissue samples to be captured within the lumen 422 with less need for suction and the consequent drawing of blood and other non-targeted tissues into the lumen 422. This motion substantially mimics a maneuver (juicing) currently performed manually by moving the entire device proximally and distally to drive the tip of a biopsy needle repeatedly into engagement with target tissue. However, the more controlled overall movement and ratio of rotation to linear advancement provided by the device 400 allows for the optimization of this motion to maximize tissue sample quality while minimizing trauma to the surrounding tissue.

A distal portion of the needle member 410 may include a plurality of surface incongruities (e.g., projections 450) extending along a path substantially parallel to a path of the threading of the portion 426 so that, as the needle member 410 is screwed into tissue, the projections 450 rotate along a path substantially similar to that of the portion 426 and, by providing echo-sonic reflection areas when used in conjunction with ultrasound imaging, indicate to a user the precise linear and rotational motion of the distal tip 414 of the needle member 410. The projections 450, in addition to serving as echo-reflectors, also help a user to visualize rotation of the needle member 410 and to provide rotation thereto as the needle member 410 slides against the inner wall 418, the projections being formed of, for example, a lubricious polymer such as TFE. Those skilled in the art will understand that the projections 450 are sized to provide a clear image of the motion of the distal end of the needle member 410 but that they preferably project from the outer surface of the needle 426 by a distance which allows the distal tip 414 to be fully withdrawn into the working channel 404 (i.e., the outer diameter of the portion of the needle member 410 including the projections 450 is less than the inner diameter of the distal portion of the working channel 404). In another embodiment of the invention the projections 450 may be formed as any surface features permitting echo-reflection (i.e., raised portions, indentations, insets, etc.). For example, indentations (not shown) provided on the needle member 410 may be formed as one continuous, threadlike spiraling indentation extending around the needle member 410 to permit a screw-like insertion of the needle member 410 into the tissue when coming into contact with a corresponding threading or abutment formed on an outer wall of the working channel 404 or as a plurality of separate indentations spaced from one another to permit an axial insertion into tissue. In another example, insets made of a material with a high echo-reflectivity may be embedded on an outer surface of the needle member 410 to provide a reflective advantage over a solid needle member 410. The insets may be provided in any patterns such as a spiral pattern selected to match a corresponding thread in the working channel 404 and permit a screw-like movement of the needle member 410 thereoutof. The surface features discussed above may be provided from a proximal end located proximally of a needle taper to a distal end of the needle member 410. In a preferred embodiment, the surface features may function as both reflecting agents and as screw guides to permit screwable insertion of the needle member 410 into a target portion of tissue.

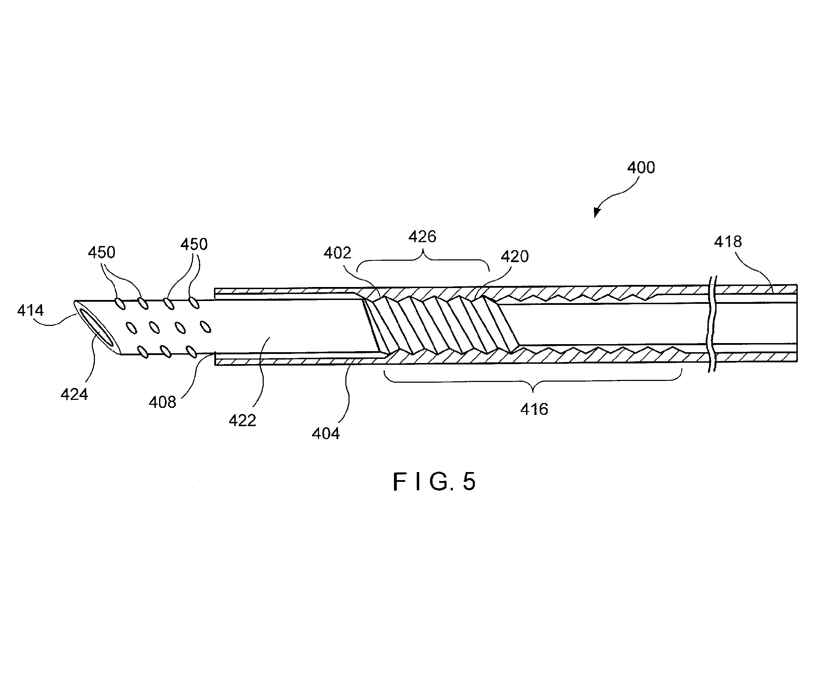

The present invention may be applied to medical and scientific procedures that require the insertion of a needle into tissue. Though the present invention has been described with respect to the retrieval of tissue samples, it is submitted that many alternate uses such as, for example, mechanisms according to this invention may be employed for the needles for injection or withdrawal of fluids may be employed without deviating from the scope of the present invention. For example, the translation of movement to a rotational mechanism as disclosed herein may be applicable to any medical device requiring the rotation of a distal tip upon actuation thereof, the medical device including, but not limited to cytology brushes, biopsy needles, snares, fluid sprayers, optic fibers, cutting tools, biopsy forceps, graspers, hooks and the like. Furthermore, although embodiments of the present invention are directed to a catheter sheet comprising an abutment and a needle having grooves, the present invention also covers embodiments wherein the abutments are formed on the needle and a groove is formed on the catheter shaft, as depicted in one embodiment in Fig. 5. Thus, it is to be understood that these embodiments have been described in an exemplary manner and are not intended to limit the scope of the invention which is intended to cover all modifications and variations of this invention that come within the scope of the appended claims

## Claims

1. A device (100, 200, 400) for penetrating tissues within a living body, comprising:
a shaft (110) extending from a proximal end to a distal end, the shaft (110) comprising a channel extending therethrough from the proximal end to an opening (424) in the distal end;
a needle (102, 202, 410) extending within the channel, the needle (102, 202, 410) comprising a proximal portion and a distal portion (108) the distal portion (108) including a tissue penetrating distal tip (104, 204), a portion of an outer surface of the distal portion (108) of the needle (102, 202, 410) including a first structure (118, 218) extending along a path wrapping around a portion of a length of the needle (102, 202), the first structure (118, 218) configured to mate with a second structure (120) formed on a corresponding portion of an inner wall of the shaft, the first and second structures (118, 218, 120) mating with one another so that, as the proximal portion of the needle (102, 202, 410) is translated axially through the channel, engagement between the first and second structures (118, 218, 120) rotates the distal portion of the needle (102, 202, 410) about an axis of the channel; **characterized in that** the proximal portion and the distal portion (108) are rotatably coupled to one another.

2. The device (100, 200, 400) according to claim 1, wherein the first structure (118, 218) is a groove and the second structure (120) is an abutment engaging the groove.

3. The device (100, 200, 400) according to claim 2, wherein the groove is substantially helical, a length of the groove along the axis being less than a length of the needle (102, 202, 410) along the axis.

4. The device (100, 200, 400) according to claim 1, wherein the shaft (110, 210) defines a shaft lumen (116, 216, 216) and the needle (102, 202, 410) defines a needle lumen (114, 214) extending to a needle opening (424) in a distal end of the needle (102, 202), the needle lumen (114, 214) and shaft lumen (116, 216, 216) being fluidly connected to one another.

5. The device (100, 200, 400) according to claim 2, wherein the shaft (110, 210) is coupled to the needle (102, 202, 410) by a hollow joint (112, 212) which fluidly couples a distal end of the shaft lumen (116, 216, 216) to a proximal end of the needle lumen (114, 214), the joint allowing the needle (102, 202, 410) to rotate freely about the axis.

6. The device (100, 200, 400) according to claim 2, wherein a length of the groove along the axis is selected to define a maximum length of the needle (102, 202, 410) which may be extended from a distal end of the catheter, the length being selected to permit the needle (102, 202, 410) to be fully retractable into the catheter.

7. The device (100, 200, 400) according to claim 2, wherein the abutment is moveable between an engaged position in which the groove is engaged and a retracted position in which the needle (102, 202, 410) is freed to move axially through the catheter without rotation about the axis.

8. The device (100, 200, 400) according to claim 7, further comprising an actuator (320) enabling a user to move the abutment between the retracted and engaged positions.

9. The device (100, 200, 400) according to claim 7, wherein the abutment is a pawl (220) rotatably coupled to an inner surface of the catheter, wherein distal surfaces of the groove are ramped to enable the pawl (220) to ratchet along an outer surface of the needle (102, 202, 410) as the needle (102, 202, 410) is withdrawn proximally into the catheter without rotating about the axis.

10. The device (100, 200, 400) according to claim 9, further comprising a biasing member (224) coupled between the catheter and the pawl (220) biasing the pawl (220) toward the engaged position.

11. The device (100, 200, 400) according to claim 8, further comprising a mechanism moving the pawl (220) to the retracted position when the needle (102, 202, 410) has reached a distal most position and releasing the pawl (220) to move back to the engaged position when the needle (102, 202, 410) is fully retracted into the catheter.

12. The device (100, 200, 400) according to claim 4, further comprising a proximal port fluidly coupled to the shaft lumen (116, 216, 216) so that, negative fluid pressure applied thereto creates suction into the needle opening (424).

13. The device (100, 200, 400) according to claim 2, wherein a pitch of the groove varies along a length of the groove, the pitch being reduced at least one of the proximal and distal ends thereof to a pitch of a central portion of the groove.

14. The device (100, 200, 400) according to claim 1, wherein a maximum outer diameter of the first structure exceeds an inner diameter of a portion of the channel distal of the second structure to define a maximum extension of the tip from the distal opening (408) of the channel.

15. The device (100, 200, 400) according to claim 14, further comprising a third structure including a plurality of surface incongruities (450) extending along a path along a portion of the needle (102, 202, 410) member which, when the needle (102, 202, 410) is extended distally from the distal opening (408) of the channel, are located distal of the distal opening, wherein the surface incongruities include one of projections from an outer surface of the needle member, indentations in the outer surface of the needle member and insets in the outer surface of the needle member of a material having echo-sonic location properties different from a material of which surrounding portions of the needle member are formed.

## Patentansprüche

1. Vorrichtung (100, 200, 400) zum Durchdringen von Geweben in einem lebenden Körper, die aufweist:
einen Schaft (110) der sich von einem proximalen Ende zu einem distalen Ende erstreckt, wobei der Schaft (110) einen Kanal aufweist, der sich dort hindurch vom proximalen Ende zu einer Öffnung (424) im distalen Ende erstreckt;
eine Nadel (102, 202, 410), die sich innerhalb des Kanals erstreckt, wobei die Nadel (102, 202, 410) einen proximalen Abschnitt und einen distalen Abschnitt (108) aufweist, wobei der distale Abschnitt (108) eine gewebedurchdringende distale Spitze (104, 204) aufweist, wobei ein Abschnitt einer Außenfläche des distalen Abschnitts (108) der Nadel (102, 202, 410) eine erste Struktur (118, 218) aufweist, die sich längs eines Wegs erstreckt, der einen Abschnitt einer Länge der Nadel (102, 202) umschlingt,
wobei die erste Struktur (118, 218) so konfiguriert ist, dass sie mit einer zweiten Struktur (120) zusammenpasst, die an einem entsprechenden Abschnitt einer Innenwand des Schafts ausgebildet ist, wobei die erste und zweite Struktur (118, 218, 120) miteinander zusammenpassen, so dass, wenn der proximale Abschnitt der Nadel (102, 202, 410) axial durch den Kanal verschoben wird, ein Eingriff zwischen der ersten und zweiten Struktur (118, 218, 120) den distalen Abschnitt der Nadel (102, 202, 410) um eine Achse des Kanals dreht;
**dadurch gekennzeichnet, dass** der proximale Abschnitt und der distale Abschnitt (108) drehbar miteinander gekoppelt sind.

2. Vorrichtung (100, 200, 400) nach Anspruch 1, wobei die erste Struktur (118, 218) eine Nut ist und die zweite Struktur (120) ein Widerlager ist, das in die Nut eingreift.

3. Vorrichtung (100, 200, 400) nach Anspruch 2, wobei die Nut im Wesentlichen helikal ist und eine Länge der Nut längs der Achse kleiner als eine Länge der Nadel (102, 202, 410) längs der Achse ist.

4. Vorrichtung (100, 200, 400) nach Anspruch 1, wobei der Schaft (110, 210) ein Schaftlumen (116, 216, 216) definiert und die Nadel (102, 202, 410) ein Nadellumen (114, 214) definiert, das sich zu einer Nadelöffnung (424) in einem distalen Ende der Nadel (102, 202) erstreckt, wobei das Nadellumen (114, 214) und das Schaftlumen (116, 216, 216) fluidisch miteinander verbunden sind.

5. Vorrichtung (100, 200, 400) nach Anspruch 2, wobei der Schaft (110, 210) mit der Nadel (102, 202, 410) durch eine Hohlverbindung (112, 212) gekoppelt ist, die ein distales Ende des Schaftlumens (116, 216, 216) mit einem proximalen Ende des Nadellumens (114, 214) koppelt, wobei es die Verbindung ermöglicht, dass sich die Nadel (102, 202, 410) frei um die Achse dreht.

6. Vorrichtung (100, 200, 400) nach Anspruch 2, wobei eine Länge der Nut längs der Achse so ausgewählt ist, dass sie eine maximale Länge der Nadel (102, 202, 410) definiert, die aus einem distalen Ende des Katheters ausgefahren werden kann, wobei die Länge so ausgewählt ist, dass es ermöglicht wird, dass die Nadel (102, 202, 410) vollständig in den Katheter einziehbar ist.

7. Vorrichtung (100, 200, 400) nach Anspruch 2, wobei das Widerlager zwischen einer Eingriffsposition, in der die Nut in Eingriff steht, und einer eingezogenen Position beweglich ist, in der die Nadel (102, 202, 410) gelöst ist, so dass sie sich axial durch den Katheter ohne eine Drehung um die Achse bewegt.

8. Vorrichtung (100, 200, 400) nach Anspruch 7, die ferner ein Bedienungselement (320) aufweist, das es einem Benutzer ermöglicht, das Widerlager zwischen der eingezogenen Position und der Eingriffsposition zu bewegen.

9. Vorrichtung (100, 200, 400) nach Anspruch 7, wobei das Widerlager eine Sperrklinke (220) ist, die drehbar mit einer Innenfläche des Katheters gekoppelt ist, wobei distale Oberflächen der Nut rampenförmig sind, um es der Sperrklinke (220) zu ermöglichen, längs einer Außenfläche der Nadel (102, 202, 410) zu rasten, wenn die Nadel (102, 202, 410) proximal in den Katheter ohne eine Drehung um die Achse eingezogen wird.

10. Vorrichtung (100, 200, 400) nach Anspruch 9, die ferner ein Vorspannelement (224) aufweist, das zwischen den Katheter und die Sperrklinke (220) gekoppelt ist, das die Sperrklinke (220) zur Eingriffsposition vorspannt.

11. Vorrichtung (100, 200, 400) nach Anspruch 8, die ferner einen Mechanismus aufweist, der die Sperrklinke (220) zur eingezogenen Position bewegt, wenn die Nadel (102, 202, 410) eine am weitesten distale Position erreicht hat, und die Sperrklinke (220) löst, um sich zurück zur Eingriffsposition zu bewegen, wenn die Nadel (102, 202, 410) vollständig in den Katheter eingezogen ist.

12. Vorrichtung (100, 200, 400) nach Anspruch 4, die ferner einen proximalen Port aufweist, der fluidisch mit dem Schaftlumen (116, 216, 216) gekoppelt ist, so dass ein darauf ausgeübter Fluidunterdruck einen Sog in die Nadelöffnung (424) erzeugt.

13. Vorrichtung (100, 200, 400) nach Anspruch 2, wobei eine Steigung der Nut längs einer Länge der Nut variiert, wobei die Steigung an deren proximalen Ende und/oder an deren distalen Ende gegenüber einer Steigung eines mittleren Abschnitts der Nut reduziert ist.

14. Vorrichtung (100, 200, 400) nach Anspruch 1, wobei ein maximaler Außendurchmesser der ersten Struktur einen Innendurchmesser eines Abschnitts des Kanals distal von der zweiten Struktur übersteigt, um eine maximale Ausdehnung der Spitze aus der distalen Öffnung (408) des Kanals zu definieren.

15. Vorrichtung (100, 200, 400) nach Anspruch 14, die ferner eine dritte Struktur aufweist, die mehrere Oberflächennichtübereinstimmungen (450) aufweist, die sich längs eines Wegs längs eines Abschnitts des Nadelelements (102, 202, 410) erstrecken, die, wenn die Nadel (102, 202, 410) distal aus der distalen Öffnung (408) des Kanals ausgefahren wird, distal von der distalen Öffnung angeordnet sind, wobei die Oberflächennichtübereinstimmungen Vorsprünge aus einer Außenfläche des Nadelelements oder Vertiefungen in der Außenfläche des Nadelelements oder Einsätze in die Außenfläche des Nadelelements aus einem Material umfassen, das echoakustische Ortungseigenschaften aufweist, die sich von einem Material unterscheiden, aus dem umgebende Abschnitte des Nadelelements ausgebildet sind.

## Revendications

1. Dispositif (100, 200, 400) pour la pénétration de tissus à l'intérieur d'un corps vivant, comprenant :
une tige (110) s'étendant d'une extrémité proximale à une extrémité distale, ladite tige (110) comprenant un canal s'étendant à l'intérieur de celle-ci de l'extrémité proximale à un orifice (424) de l'extrémité distale ;
une aiguille (102, 202, 410) s'étendant à l'intérieur du canal, ladite aiguille (102, 202, 410) comprenant une partie proximale et une partie distale (108) ;
la partie distale (108) comprenant pointe distale (104, 204) de pénétration du tissu, une partie d'une surface extérieure de la partie distale (108) de l'aiguille (102, 202, 410) présentant une première structure (118, 218) s'étendant sur un trajet enroulé autour d'une partie de la longueur de l'aiguille (102, 202), ladite première structure (118, 218) étant prévue pour coïncider avec une deuxième structure (120) formée sur une partie correspondante d'une paroi intérieure de la tige, la première et la deuxième structures (118, 218, 120) coïncidant l'une avec l'autre de telle manière que, lorsque la partie proximale de l'aiguille (102, 202, 410) est translatée axialement par le canal, l'engrènement entre la première et la deuxième structures (118, 218, 120) fait tourner la partie distale de l'aiguille (102, 202, 410) autour de l'axe du canal ;
**caractérisé en ce que** la partie proximale et la partie distale (108) sont solidaires en rotation entre elles.

2. Dispositif (100, 200, 400) selon la revendication 1, où la première structure (118, 218) est une rainure et la deuxième structure (120) une butée s'engageant dans la rainure.

3. Dispositif (100, 200, 400) selon la revendication 2, où la rainure est sensiblement hélicoïdale, la longueur de la rainure le long de l'axe étant inférieure à la longueur de l'aiguille (102, 202, 410) le long de l'axe.

4. Dispositif (100, 200, 400) selon la revendication 1, où la tige (110, 210) définit une lumière (116, 216, 216) de tige et l'aiguille (102, 202, 410) définit une lumière (114, 214) d'aiguille s'étendant vers un orifice (424) d'aiguille à une extrémité distale de l'aiguille (102, 202), la lumière (114, 214) d'aiguille et la lumière (116, 216, 216) de tige étant en communication fluidique l'une avec l'autre.

5. Dispositif (100, 200, 400) selon la revendication 2, où la tige (110, 210) est reliée à l'aiguille (102, 202, 410) par un joint creux (112, 212) reliant fluidiquement une extrémité distale de la lumière (116, 216, 216) de tige à une extrémité proximale de la lumière (114, 214) d'aiguille, ledit joint permettant à l'aiguille (102, 202, 410) de tourner librement autour de l'axe.

6. Dispositif (100, 200, 400) selon la revendication 2, où la longueur de la rainure le long de l'axe est sélectionnée pour définir une longueur maximale de l'aiguille (102, 202, 410) pouvant s'étendre depuis l'extrémité distale du cathéter, ladite longueur étant sélectionnée pour permettre à l'aiguille (102, 202, 410) d'être entièrement rétractée dans le cathéter.

7. Dispositif (100, 200, 400) selon la revendication 2, où la butée est mobile entre une position d'engrènement où la rainure est occupée et une position de rétraction où l'aiguille (102, 202, 410) est libérée pour être mobile axialement le long du cathéter sans tourner autour de l'axe.

8. Dispositif (100, 200, 400) selon la revendication 7, comprenant en outre un actionneur (320) permettant à un utilisateur de déplacer la butée entre la position de rétraction et la position d'engrènement.

9. Dispositif (100, 200, 400) selon la revendication 7, où la butée est un cliquet (220) solidaire en rotation avec une surface intérieure du cathéter, où des surfaces distales de la rainure sont inclinées pour permettre au cliquet (220) de glisser sur une surface extérieure de l'aiguille (102, 202, 410) lorsque l'aiguille (102, 202, 410) est rétractée proximalement dans le cathéter sans tourner autour de l'axe.

10. Dispositif (100, 200, 400) selon la revendication 9, comprenant en outre un élément de contrainte (224) raccordé entre le cathéter et le cliquet (220), repoussant le cliquet (220) vers la position d'engrènement.

11. Dispositif (100, 200, 400) selon la revendication 8, comprenant en outre un mécanisme déplaçant le cliquet (220) vers la position de rétraction lorsque l'aiguille (102, 202, 410) a atteint une position distale extrême, et relâchant le cliquet (220) pour retourner à la position d'engrènement lorsque l'aiguille (102, 202, 410) est entièrement rétractée dans le cathéter.

12. Dispositif (100, 200, 400) selon la revendication 4, comprenant en outre un orifice proximal communiquant fluidiquement avec la lumière (116, 216, 216) de tige de telle manière qu'une pression fluidique négative appliquée génère une aspiration dans l'orifice (424) d'aiguille.

13. Dispositif (100, 200, 400) selon la revendication 2, où un pas de la rainure varie sur la longueur de la rainure, le pas diminuant depuis l'extrémité proximale et/ou l'extrémité distale de celle-ci vers le pas d'une partie centrale de la rainure.

14. Dispositif (100, 200, 400) selon la revendication 1, où le diamètre extérieur maximal de la première structure est supérieur au diamètre intérieur d'une partie du canal distal de la deuxième structure pour définir une extension maximale de la pointe depuis l'orifice distal (408) du canal.

15. Dispositif (100, 200, 400) selon la revendication 14, comprenant en outre une troisième structure présentant une pluralité de déformations (450) de surface s'étendant sur un trajet le long d'une partie de l'élément d'aiguille (102, 202, 410), lesquelles sont situées distalement par rapport à l'orifice distal lorsque l'aiguille (102, 202, 410) a une extension distale depuis l'orifice distal (408) du canal, les déformations de surface comprenant soit des saillies sur une surface extérieure de l'élément d'aiguille, soit des renfoncements dans la surface extérieure de l'aiguille, soit des inserts dans la surface extérieure de l'élément d'aiguille en matériau présentant des propriétés échosonores de positionnement différentes de celles du matériau dont sont formées les parties environnantes de l'élément d'aiguille.
